# EUROPEAN PATENT SPECIFICATION

(11) **EP 4 059 483 B1**
(45) Date of publication and mention of the grant of the patent: **14.10.2026**
(21) Application number: 21163723.6
(22) Date of filing: 19.03.2021
(51) Int. Cl.: A61F 13/15, A61F 13/62

(54) **A TRANSVERSELY EXTENSIBLE CONTINUOUS ELASTIC LAMINATE, AND A METHOD FOR PRODUCING THE SAME**
QUER DEHNBARES, KONTINUIERLICHES, ELASTISCHES LAMINAT UND VERFAHREN ZUR HERSTELLUNG DAVON
STRATIFIÉ ÉLASTIQUE CONTINU EXTENSIBLE TRANSVERSALEMENT ET SON PROCÉDÉ DE FABRICATION

(43) Date of publication of application: 21.09.2022
(73) Proprietor: Fameccanica.Data S.p.A., 66020 San Giovanni Teatino (CH) (IT)
(72) Inventor: GUALTIERI, Diego, I-66020 San Giovanni Teatino (Chieti) (IT); CIPRIANI, Alessandro, I-66020 San Giovanni Teatino (Chieti) (IT)
(74) Representative: Buzzi, Notaro & Antonielli d'Oulx S.p.A.

(56) References cited:
- EP-A1- 3 213 728

## Description

### Field of the invention

The present invention relates to a transversely extensible continuous elastic laminate.

The present invention has been developed with particular reference to the production of disposable absorbent sanitary articles, such as, for example, diapers, training pants, absorbent sanitary products for incontinent adults, etc.

A transversely extensible continuous elastic laminate may constitute a semi-finished intermediate product which can be used for supplying machines for manufacturing absorbent sanitary articles. The transversely extensible continuous elastic laminate can be cut transversely to produce elastic side panels for absorbent sanitary articles. Discrete left and right fastening tabs could be already attached externally on the elastic laminate.

Embodiments of the present invention also relate to an elastic side panel for absorbent sanitary articles.

The present invention also relates to methods for producing transversely extensible continuous elastic laminates.

### Prior art

An absorbent sanitary article wearable as a pant typically has a structure that comprises a rectangularshaped central body or chassis and at least one pair of elastic side panels which extend laterally from opposite sides of the central body in the front or rear section of the central body. The side panels are provided with fastening tabs, typically comprising micro-hooks pads, which can be releasably attached to a front panel for closing the absorbent sanitary article around the waist of the user.

EP-A-3213728 discloses a transversely extensible elastic laminate comprising: a first and a second web, having respective pleated central portions, an elastic tape sandwiched between the pleated central portions of the first and second web and elastically stretchable in a transverse direction, and a plurality of fastening tabs spaced apart from each other in a longitudinal direction.

The fastening tabs each include a non-woven support web of soft material and a micro-hook pad of relatively rigid thermoplastic material fixed on a surface of the non-woven support web by glue or by welding.

The micro-hook pads are produced starting from continuous tapes, usually made of relatively rigid thermoplastic material, having continuous or intermittent micro-hook formations integrally formed thereon. The continuous tape with integrally formed micro-hook formations is cut to form a plurality of discrete micro-hook pads. Such discrete micro-hook pads are spaced by a desired pitch and are fixed in spaced positions on a surface of a continuous non-woven support web. The continuous non-woven support web with the micro-hook pads fixed thereon is then cut to form fastening tabs each including a non-woven support web of soft material and a micro-hook pad of relatively rigid thermoplastic material fixed on a surface of the non-woven support web by glue or by welding.

The continuous tapes with integrally formed micro-hook formations is manufactured by suppliers specialized in manufacturing hook-and-loop fasteners. The micro-hook tapes are packaged in reels which are delivered to the manufacturing plants of absorbent sanitary articles. In the machines for manufacturing absorbent sanitary articles the reels of continuous tapes with integrally formed micro-hook formations are unwound, cut, spaced, and fixed to the continuous non-woven support web. All these operations require complex and expensive dedicated equipment which increase the complexity of the machines for manufacturing absorbent sanitary articles. The cost of the continuous tapes with integrally formed micro-hook formations is high, therefore, the dimensions of the micro-hook pads or patches is kept to the minimum possible, which requires complex equipment for cutting the continuous tapes with integrally formed micro-hook formations in very small pieces and for feeding such small pieces with high precision to the continuous non-woven support web.

A further drawback of the prior art is that the micro-hook fastening pads extend for the whole width of the support tape, i.e., the lateral edges of the micro-hook pads are aligned to the lateral edges of the support web. When touching the fastening tab, the user perceives a sharp feeling due to the fact that the rigid micro-hook pads extend up to the edges of the fastening tab. This structure of the fastening tabs gives rise to an unpleasant stiffness and sharpness perception during the application of the sanitary article to the wearer. It is not unusual that the user gets scratches or small cuts on the soft skin under the nails due to the contact with sharp and rigid edges of the micro-hook pads.

### Object and summary of the invention

The object of the present invention is to provide a transversely extensible continuous elastic laminate usable to produce elastic side panels for absorbent sanitary articles, which overcomes the problem of the prior art.

According to a first aspect of the invention, this object is achieved by a transversely extensible continuous laminate having the features of claim 1.

An important feature of the present invention is that the tabs with micro-hook pads can be manufactured in-line in a machine for manufacturing absorbent sanitary articles starting from reels of non-woven material. Therefore, there is no need to supply the machine for manufacturing absorbent sanitary articles with reels of micro-hook tapes supplied by companies specialized in manufacturing hook-and-loop fasteners.

The present invention eliminates handling and transportation of micro-hook tapes, which reduces costs, simplifies handling of raw material in the plants for manufacturing absorbent sanitary articles, and has a positive impact on sustainability due to the elimination of the need of transporting the micro-hook tapes.

According to another aspect, the present invention relates to an elastic side panel for absorbent sanitary articles having the features of claim 6.

According to another aspect, the invention relates to methods for producing transversely extensible laminates having the features of claim 10, 11 and 12.

The manufacturing methods, as compared to the prior art, provide a better control on the distance between the fastening tabs, which in the method according to the invention are not applied as discontinuous discrete elements. This reduces manufacturing wastes and has a direct positive impact on sustainability.

The claims form an integral part of the technical disclosure provided here in relation to the invention.

### Brief description of the drawings

The present invention will now be described with reference to the attached drawings, provided purely by way of non-limiting example, wherein:
- figures 1-10 are schematic views showing various steps of a first embodiment of a method for producing a transversely extensible continuous elastic laminate having integral micro-hook pads,
- figure 11 is a schematic plan view of a transversely extensible continuous elastic laminate in an extended position,
- figure 12 is a schematic cross-section taken along the line XII-XII of figure 11,
- figure 13 is a schematic plan view showing the transversely extensible continuous elastic laminate of figure 11 in a folded configuration,
- figure 14 is a schematic cross-section taken along the line XIV-XIV of figure 13,
- figure 15 is a schematic plan view showing an elastic side panel for absorbent sanitary articles in an extended position,
- figure 16 is a schematic plan view showing an elastic side panel with a different shape,
- figures 17-20 are schematic plan views showing various steps of a second embodiment of a method for producing a transversely extensible continuous elastic laminate having integral micro-hook pads,
- figure 21 is a schematic plan view of a second embodiment of a transversely extensible continuous elastic laminate in an extended position,
- figure 22 is a schematic cross-section taken along the line XXII-XXII of figure 21,
- figures 23-29 are schematic plan views showing various steps of a third embodiment of a method for producing a transversely extensible continuous elastic laminate having integral micro-hook pads,
- figure 30 is a schematic plan view of a third embodiment of a transversely extensible continuous elastic laminate in an extended position, and
- figure 31 is a schematic cross-section taken along the line XXXI-XXXI of figure 30.

In the following description, identical or similar components will be indicated by the same reference numerals.

It should be appreciated that the attached drawings are schematic and not to scale with respect to real products. Various figures may not be represented in the same scale. Also, in various figures some elements may not be shown to better show other elements.

### Detailed description

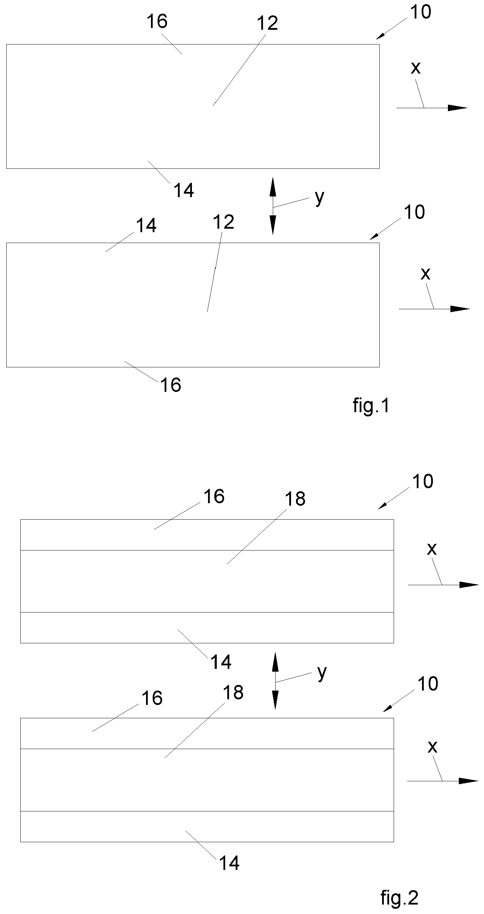

Figures 1-10 schematically show various steps of a method for producing a transversely extensible continuous elastic laminate.

In a first step, shown in figure 1, two first continuous webs 10 are fed in a longitudinal direction X. The two first continuous webs 10 may be made of a non-woven material. In a possible embodiment, the two first continuous webs 10 may be obtained from a single continuous web unwound from a reel and cut longitudinally so as to form two separate webs advancing in the longitudinal direction X.

The two first continuous webs 10 are spaced apart from each other in a transversal direction Y orthogonal to the longitudinal direction X.

Each of the two first continuous webs 10 has a central portion 12 and first and second edge portions 14, 16 opposite to each other with respect to the central portion 12.

The two first continuous webs 10 may be positioned on the outer surface of a wheel or on the upper surface of a conveyor belt as they advance continuously in the longitudinal direction X.

In a second step, shown in figure 2, two continuous elastic tapes 18 are fed in the same longitudinal direction X and are overlapped to the respective central portions 12 of the two first continuous webs 10. The two elastic tapes 18 may be obtained from a single continuous elastic tape unwound from a reel and cut longitudinally so as to form the two separate elastic tapes advancing in the longitudinal direction X.

The two elastic tapes 18 are elastically stretched in the transversal direction Y when they are applied to the respective central portions 12 of the two continuous webs 10. The two continuous elastic tapes 18 may have the capability to extend in the transversal direction Y by at least 100%, and preferably by 300%, with respect to the respective rest dimensions and to return to the respective rest dimensions in the absence of a transversal force. In a possible embodiment, the degree of transversal elastic stretching of the two continuous elastic tapes 18 may be in the order of 200%. This means that the two continuous elastic tapes 18 are applied on the respective first continuous webs 10 with a width essentially equal to three times the width that the continuous elastic tapes 18 have at rest, i.e., in the absence of transversal forces.

The transverse extension of the two continuous elastic tapes 18 may be obtained by a spreading device comprising, for each continuous elastic tape 18, two wheels with respective axes inclined with respect to each other, as disclosed in EP-A-3213728.

The transversely stretched continuous elastic tapes 18, once applied to the respective central portions 12 of the two first continuous webs 10 may be retained in the stretched state by vacuum suction, as disclosed in EP-A-3213728.

In a possible embodiment, the continuous elastic tapes 18 may have different widths in the transverse direction Y and/or may be stretched in said transverse direction Y to reach different widths.

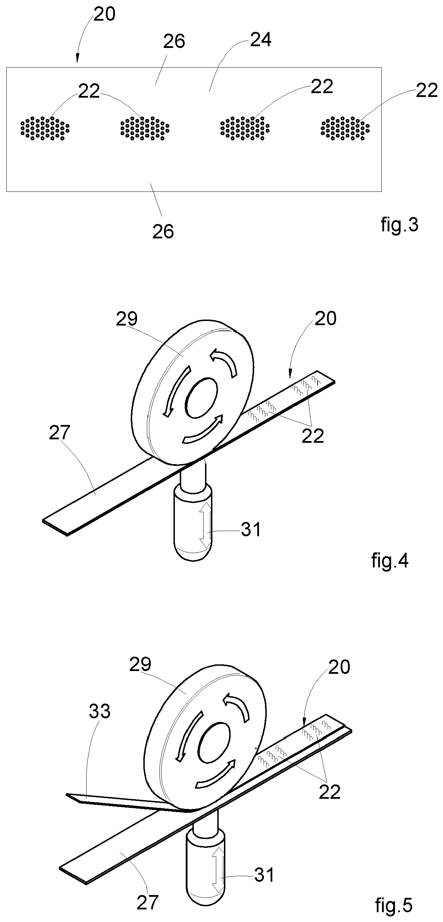

With reference to figure 3, the method comprises providing a continuous support web 20 made of a non-woven material and having a row of micro-hook pads 22 integrally formed on a planar surface 24 of the support web 20.

The continuous support web 20 has two continuous longitudinal edge portions 26 and the row of micro-hook pads 22 is located in a central portion of the support web 20. The micro-hook pads 22 are spaced apart from each other in the longitudinal direction X.

In a possible embodiment, the micro-hook pads 22 may have the respective micro-hooks alternately oriented in opposite directions, so that in each pair of adjacent micro-hook pads 22 the micro-hooks of one pad 22 are oriented in a direction opposite with respect to the micro-hooks of the other pad 22.

The continuous support web 20 with the integrally formed micro-hook pads 22 may be formed as disclosed in detail in WO2010/085492 A1 and as shown schematically in figure 4. With reference to figure 4, a smooth non-woven web 27 passes through a micro-hook forming unit 28. The micro-hook forming unit 28 may comprise a molding roller 29 having a plurality of cavities and a ultrasonic horn 31 which compresses the non-woven web 27 against the surface of the molding roller 29.

The vibrating energy produced by the ultrasonic horn 31 liquifies or fluidifies locally the non-woven material of the non-woven web 27, which penetrates in a fluid or liquid state in the cavities of the molding roller 29. The material of the non-woven web 27 which penetrates in the cavities of the molding roller 29 after cooling forms micro-hook pads 22 integral with the non-woven material. At the exit of the micro-hook forming unit 28 a continuous support web 20 is formed having a row of integral micro-hook pads 22.

The discrete micro-hook pads 22 may be obtained by providing on the molding roller 29 an intermittent pattern of cavities. Alternatively, the molding roller 29 may have a continuous pattern of cavities and the discrete micro-hook pads 22 may be formed by intermittently stopping the power supplied to the ultrasonic horn 32. As a further alternative, the molding roller 29 may have a continuous pattern of cavities and the discrete micro-hook pads 22 may be formed by intermittently detaching the ultrasonic horn 32 from the molding roller 29.

With reference to figure 5, in a possible embodiment, an additional web 33 may be applied on the central portion of the non-woven web 27 before passing the non-woven web 27 through the micro-hook forming unit 28. The additional web 33 may be a thermoplastic film or a non-woven web of a material different or identical to the material of the non-woven web 27. After passing through the micro-hook forming unit 28 the additional web 33, the micro-hook pads 22 and the non-woven web 27 form an integral structure.

In a possible embodiment, the micro-hook forming unit 28 may be a thermomechanical forming unit, in which the material of the non-woven web 27 is locally liquified or fluidified by heating and compression.

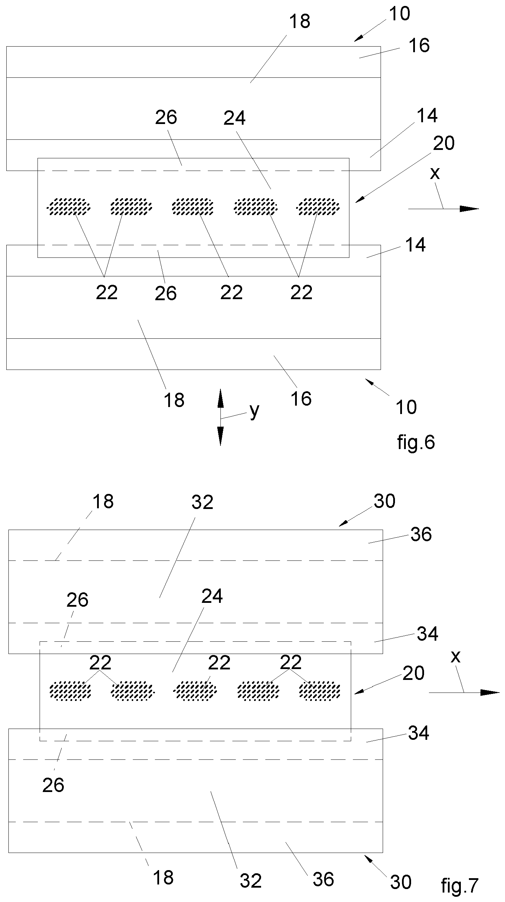

With reference to figure 6, the continuous support web 20 with integrally formed micro-hook pads 22 is fed in the longitudinal direction X and the two continuous longitudinal edge portions 26 of the support web 20 are overlapped to respective first edge portions 14 of the two first continuous webs 10. The two continuous longitudinal edge portions 26 of the continuous support web 20 may be retained on the respective edge portions 14 of the two first continuous webs 10 by the same vacuum suction which holds the stretched elastic tapes 18 on the first webs 10.

As shown in figure 7, in a further step two second continuous webs 30 are fed in the longitudinal direction Y, and are overlapped to the respective first continuous webs 10.

In a possible embodiment, the two second continuous webs 30 may be obtained from a single continuous web unwound from a reel and cut longitudinally so as to form two separate webs advancing in the longitudinal direction X.

The two second continuous webs 30 are spaced from each other in the transverse direction Y and have respective central portions 32 and respective first and second edge portions 34, 36 on opposite sides of the central portion 32. The two second continuous webs 30 may have the same width as the first continuous webs 10. The two second continuous webs 30 are applied over the respective first continuous webs 10 so that the two continuous elastic tapes 18 are sandwiched between the respective central portions 12, 32 of the first and second webs 10, 30. Also, the two continuous longitudinal edge portions 26 of the support web 20 are sandwiched between respective first edge portions 14, 34 of the two first and second continuous webs 10, 30.

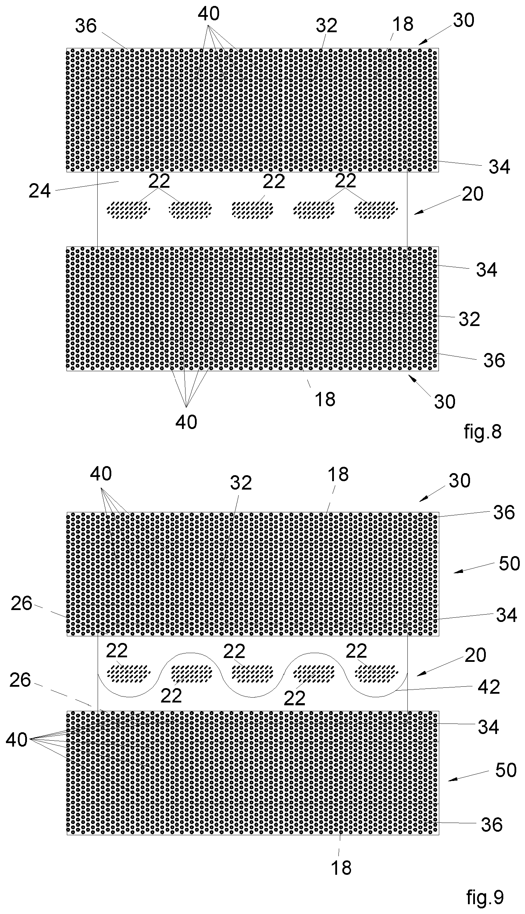

Then, as shown in figure 8, the two first and second webs 10, 30, the two elastic tapes 18 and the edge portions 26 of the support web 20 are fixed to each other by a pattern of spot welds 40. The spot welds 40 may be formed by ultrasonic welding as disclosed in EP-A-3213728. The pattern of spot welds 40 may have a reinforced area along the longitudinal portion connecting the first edge portions 14, 34 of the first and second webs to the edge portions 26 of the support web 20, in order to provide an increased joining strength in that area.

The elastic tapes 18 are stretched in the transversal direction Y during the spot welding which joins the elastic tapes 18 to the first and second webs 10, 30. The welding spots 40 may form through holes in the elastic tapes 18 in order to provide breathability features to the composite laminate, as disclosed in EP-A-3213728.

With reference to figure 9, the method further comprises a cutting step wherein the support web 20 is cut along a continuous undulated cutting line 42 passing between each pair of adjacent micro-hook pads 22. The continuous undulated cutting line 42 may be made by a cutting roller having a shaped cutting blade. The continuous undulated cutting line 42 is distanced from the edges of the micro-hook pads 22 so that each micro-hook pad 22 is completely surrounded by a portion of the planar surface 24 of the support web 20.

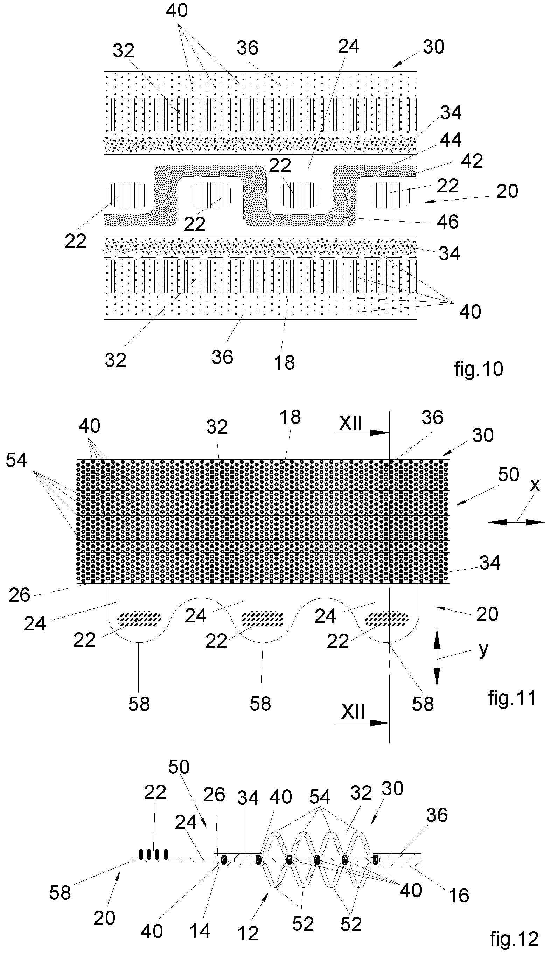

As shown in figure 10, in a possible embodiment, the support web 20 may be cut along two continuous undulated cutting lines 42, 44 passing between each pair of adjacent micro-hook pads 22. Two continuous undulated cutting lines 42, 44 define a continuous undulated strip 46 comprised between the two cutting lines 42, 44 which is disposed as waste.

The continuous undulated cutting line 42 or the continuous undulated cutting lines 42, 44 may pass at a distance from the edges of said micro-hook pads 22 comprised between 1-10 mm and preferably comprised between 2-7 mm.

After the cut of the support web 20 along the cutting line 42 or cutting lines 42, 44, two separate transversely extensible continuous elastic laminates 50 are formed, each of which has the form shown in figures 11 and 12.

With reference to figures 11 and 12, the transversely extensible continuous laminate 50 obtained by the previously disclosed method comprises a first and second continuous web 10, 30 extending along a longitudinal direction X and a continuous elastic tape 18 elastically extensible in a transversal direction Y and sandwiched between the central portions 12, 32 of the first and second continuous web 10, 30 and joined thereto by a pattern of spot welds 40.

When the vacuum suction which holds the elastic tape 18 stretched in the transversal direction Y is released, the elastic tape 18 contracts in the transversal direction Y. The transversal contraction of the elastic tape 18 forms longitudinal pleats 52, 54 on the central portions 12, 34 of the first and second continuous web 10, 30. The edge portions 14, 34 and 16, 36 of the first and second continuous webs 10, 30 which extend transversally beyond the elastic tape 18 remain non-pleated.

The transversely extensible continuous elastic laminate 50 comprises a support web 20 made of non-woven material, having a continuous longitudinal edge portions 26 which is joined to the first non-pleated edge portions 14, 34 of the first and second web 10, 30 by said pattern of spot welds 40.

The continuous longitudinal edge portion 26 of the support web 20 may be sandwiched between the first edge portions 14, 34 of the first and second web 10, 30. In a possible embodiment, the continuous longitudinal edge portion 26 of the support web 20 may be fixed on the outer surface of one of the non-pleated edge portions 14, 34 of the first and second web 10, 30.

The pattern of spot welds 40 may be reinforced in the area joining the first non-pleated edge portions 14, 34 of the first and second web 10, 30 and the continuous edge portion 26 of the support web 20.

The support web 20 includes a row of tabs 58 made of non-woven material which extend from the continuous longitudinal edge portion 26. The tabs 58 are connected to each other by the continuous edge portion 26 and are spaced apart from each other in the longitudinal direction X. Each tab 58 has a respective micro-hook pad 22 integrally formed on a planar surface 24 of the respective tab 58. The planar surface 24 of each of the tabs 58 has a portion which completely surrounds the respective micro-hook pad 22. In a possible embodiment the portion of the planar surface 24 which surrounds the respective micro-hook pad 22 may have a width greater than 2 mm. The width of each tab 58 along a line parallel to the direction X and passing through the respective micro-hook pad 22 should be at least 4 mm larger than the width of the micro-hook pad 20 along the same line (2 mm on each side of the micro-hook pad 20). The width of each tab 58 may be 100 mm larger than the width of the micro-hook pad 20. For instance, if the width of the micro-hook pad 20 is 50 mm, the width of the respective tab 58 may be comprised between 54 mm and 150 mm.

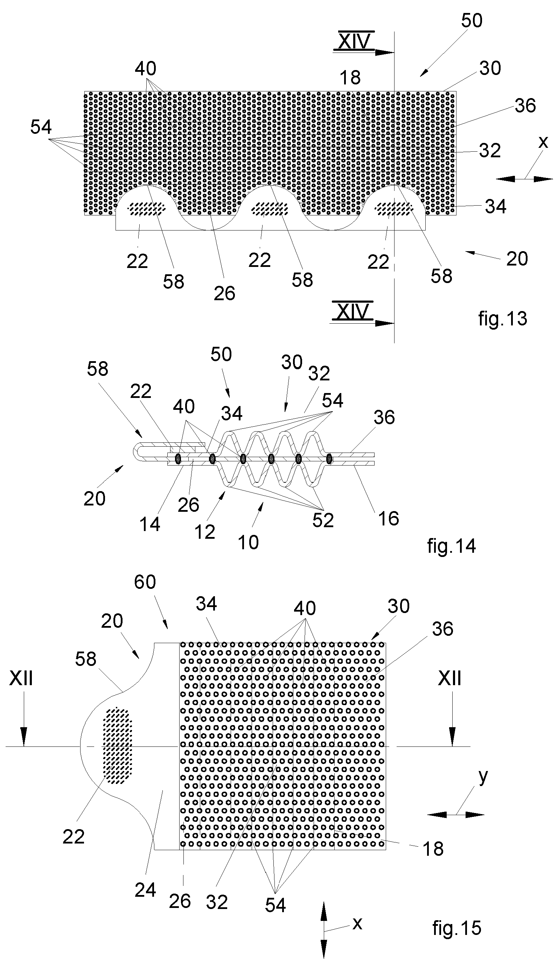

With reference to figures 13 and 14, the tabs 58 of the support web 20 may be folded over the second web 30 along a folding line parallel to the longitudinal axis X, so that the micro-hook pads 22 are releasably fastened to corresponding portions of the second web 30. Folding of the tabs 58 may be carried out before or after releasing the vacuum suction which keeps the elastic tape 18 stretched in the transverse direction Y. When the tabs 58 are folded as shown in figures 10 and 11, the transversely extensible continuous elastic laminate 50 can be handled more easily without the risk that the micro-hook pads 22 engage with a non-woven web when the transversely extensible continuous elastic laminate 50 is wound in a reel or during the manufacturing process of absorbent sanitary products.

The transversely extensible continuous elastic laminate 50 is used for producing elastic side panels in machines for manufacturing absorbent sanitary articles. The transversely extensible continuous elastic laminate 50 may be formed in-line with respect to the manufacturing machine. In a possible embodiment, the transversely extensible continuous elastic laminate 50 can be wound in reels which can be stored before being delivered to a manufacturing machine.

In the machine for manufacturing absorbent sanitary products, the transversely extensible continuous elastic laminate 50 is cut along lines generally transversal to the longitudinal axis X for forming individual elastic side panels. The cutting lines which form the individual elastic side panels may have different shapes so as to form elastic side panels with the desired shape. The cuts which originate the individual elastic side panels are typically carried out while the tabs 58 of the transversely extensible continuous elastic laminate 50 are folded as shown in figures 13 and 14.

An individual elastic side panel obtained from a transversely extensible continuous elastic laminate 50 is shown in figure 15 and is indicated by the reference numeral 60. The elastic side panel 60 is shown in an extended position for a better understanding. Figure 12, which is a cross-section taken along the line XII-XII of the transversely extensible continuous elastic laminate 50 of figure 11, is identical to a cross-section taken along the line XII-XII of the individual elastic side panel 60 of figure 15.

With reference to figures 15 and 12, the elastic side panel 60 comprises a first and a second web 10, 30 having respective pleated central portions 12, 34 having respective pleats 52, 54 parallel to a first direction X, and respective first and second non-pleated edge portions 14, 34 and 16, 36 on opposite sides of the pleated central portions 12, 32. An elastic tape 18 is sandwiched between the pleated central portions 12, 32 of the first and second web 10, 30. The elastic tape 18 is elastically stretchable in a second direction Y transversal to the first direction X. The elastic tape 18 is joined to the first and second web 10, 30 by a pattern of spot welds 40.

The elastic side panel 60 comprises a support web 20 of a non-woven material including an edge portion 26 joined to the first non-pleated edge portions 14, 34 by said pattern of spot welds 40. The pattern of spot welds 40 may be reinforced in the area connecting the first non-pleated portions 14, 34 of the first and second web 10, 30 to the edge portion 26 of the support web 20. The edge portion 26 of the support web 30 in the first direction X has the same length as the non-pleated edge portions 14, 34 of the first and second web 10, 30. The support web 20 includes a tab of non-woven material 58 extending from the edge portion 26. The tab 58 has a micro-hook pad 22 integrally formed with the non-woven material forming the tab 58. The planar surface 24 of the tab has a portion which completely surrounds the micro-hook pad 22. The portion of the planar surface 24 which surrounds the micro-hook pad 22 may have a width comprised between 1-10 mm and preferably comprised between 2-7 mm.

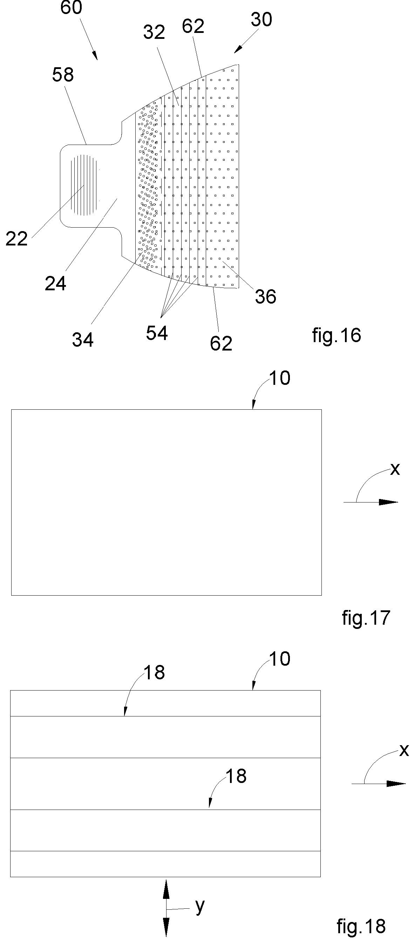

With reference to figure 16, the elastic side panel 60 may have curved flanks 62, which may be formed by cutting the transversely extensible continuous elastic laminate 50 along substantially V-shaped curved cutting lines. The portion of the transversely extensible continuous elastic laminate 50 comprised between each of the two substantially V-shaped curved cutting lines is disposed as scrap.

Figure 17-20 show a second embodiment of a method for producing a transversely extensible continuous elastic laminate. The elements corresponding to those previously disclosed are indicated by the same reference numbers.

With reference to figure 17, a first continuous web 10 is fed in a longitudinal direction X. The first continuous web 10 may be made of a non-woven material.

In a second step, shown in figure 18, two continuous elastic tapes 18 parallel to each other and spaced apart in a transversal direction Y are fed in the longitudinal direction X and are overlapped to respective portions of the first continuous web 10. The two elastic tapes 18 may be obtained from a single continuous elastic tape unwound from a reel and cut longitudinally so as to form the two separate elastic tapes advancing in the longitudinal direction X.

The two continuous elastic tapes 18 are elastically stretched in the transversal direction Y when they are applied to the continuous web 10. Transverse stretching and retention of the two elastic tapes 18 on the first continuous web 10 are carried out as previously disclosed.

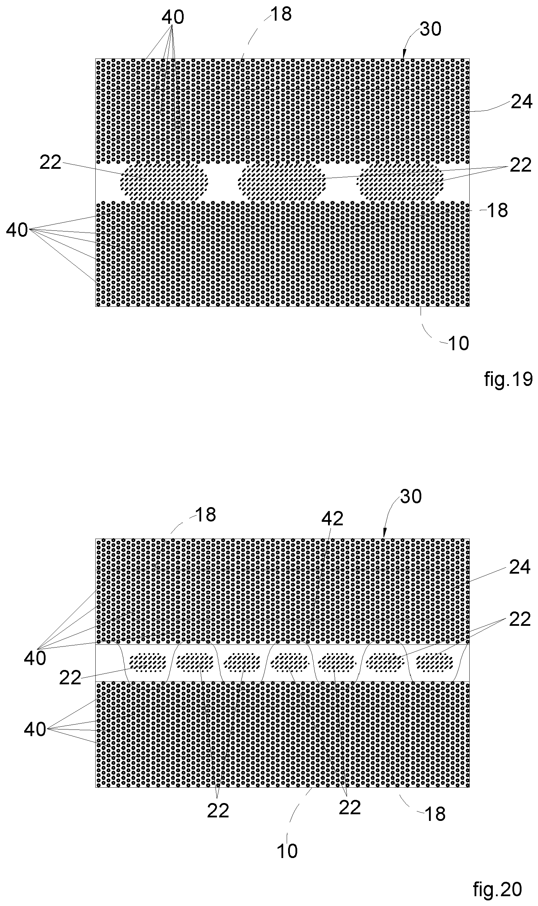

With reference to figure 19, a second continuous web 30 of a non-woven material is fed in the longitudinal direction Y and is overlapped to the first continuous web 10.

The second continuous web 30 has a row of micro-hook pads 22 integrally formed in the non-woven material forming the second continuous web 30. The row of micro-hook pads 22 is formed in a central portion of a planar surface 24 of the second continuous web 30 located between the two continuous elastic tapes 18. The micro-hook pads 22 may be formed as previously disclosed.

The two continuous elastic tapes 18 are sandwiched between respective portions of the first and second webs 10, 30.

The first and second web 10, 30, the two elastic tapes 18 are fixed to each other by a pattern of spot welds 40. The spot welds 40 may also extend through the micro-hook pads 22. The elastic tapes 18 are stretched in the transversal direction Y during the spot welding which joins the elastic tapes 18 to the first and second web 10, 30.

With reference to figure 20, the method further comprises a cutting step wherein the first and second webs 10, 30 are cut along a continuous undulated cutting line 42. The cutting line 42 passes between each pair of adjacent micro-hook pads 22 and does not cut the two elastic tapes 18.

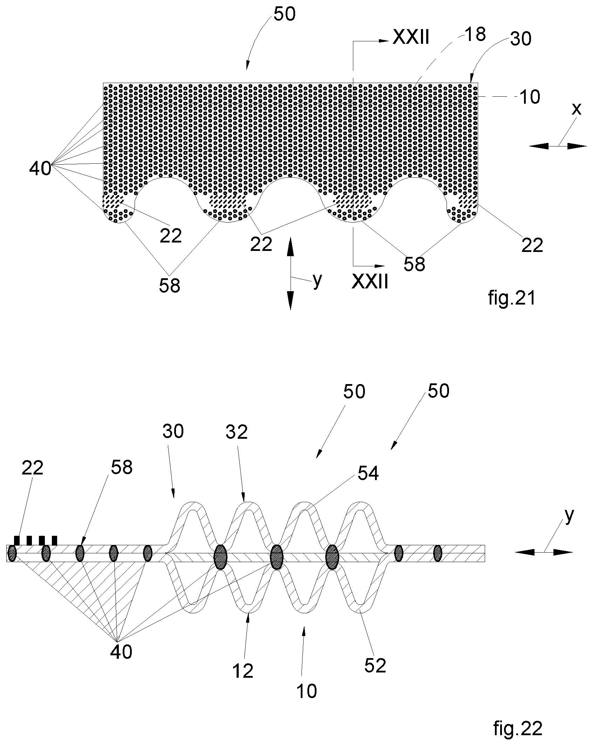

After the cut of the first and second web 10, 30 along the cutting line 42, two separate transversely extensible continuous elastic laminates 50 are formed, each of which has the form shown in figures 21 and 22.

With reference to figures 21 and 22, the transversely extensible continuous elastic laminate 50 comprises a first and a second continuous web 10, 30 extending along a longitudinal direction X and a continuous elastic tape 18 elastically extensible in a transversal direction Y and sandwiched between pleated central portions 12, 32 of the first and second continuous web 10, 30 and joined thereto by a pattern of spot welds 40.

The transversely extensible continuous elastic laminate 50 includes a row of tabs 58 of non-woven material integrally formed with the first and second continuous web 10, 30. The tabs 58 extend in the transverse direction from the pleated central portions 12, 32 and are spaced apart from each other in the longitudinal direction X.

The tabs 58 have respective micro-hook pads 22 integrally formed with the non-woven material forming the second web 30. Each micro-hook pad 22 is completely surrounded by a portion of the planar surface of the second web 30.

The tabs 58 may be folded over the second web 30 along a folding line parallel to the longitudinal axis X, so that the micro-hook pads 22 are releasably fastened to corresponding portions of the second web 30.

The transversely extensible continuous elastic laminate 50 may be cut transversely to form individual elastic side panels as previously disclosed. Each elastic side panel has a respective tab 58 integrally formed with the first and/or second non-woven webs 10, 30 and has a micro-hook pad 22 integrally formed with the non-woven material of one the two non-woven webs. The micro-hook pad 22 is completely surrounded by a portion of the planar surface of one the two non-woven webs.

Each tab 58 may be formed by two webs 10, 30 overlapped and joined to each other by a pattern of spot welds 40. The pattern of spot welds 40 may be reinforced on the tab 58. This structure makes the tab 58 particularly resistant. In a possible embodiment, each tab 58 may be formed by a single web 10 or 30 of non-woven material.

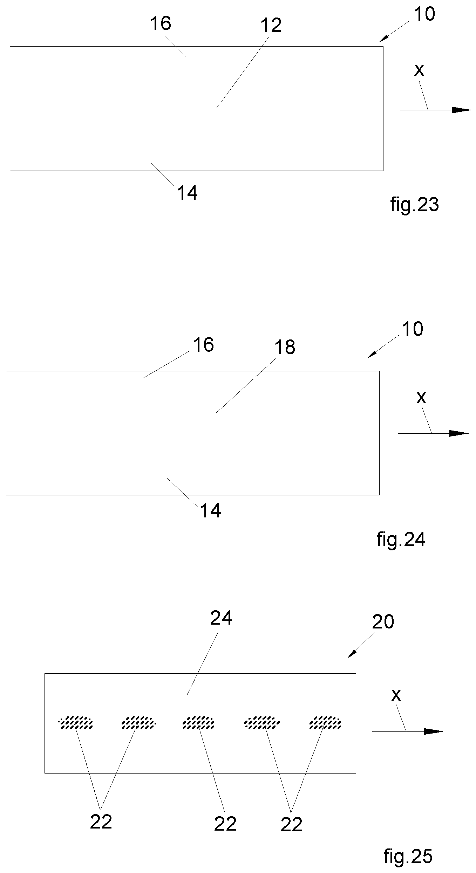

Figure 23-32 show a third embodiment of a method for producing a transversely extensible continuous elastic laminate. The elements corresponding to those previously disclosed are indicated by the same reference numbers.

With reference to figure 23 a first continuous web 10 is fed in a longitudinal direction X. The first continuous web 10 may be made of a non-woven material. The first continuous webs 10 has a central portion 12 and first and second edge portions 14, 16 opposite to each other with respect to the central portion 12.

With reference to figure 24, a continuous elastic tape 18 is fed in the same longitudinal direction X and is overlapped to the central portion 12 of the first continuous web 10. The elastic tape 18 is elastically stretched in a direction Y transversal to the longitudinal direction X when it is applied to the central portion 12 of the first continuous web 10.

With reference to figure 25, the method comprises providing a continuous support web 20 of non-woven material having an integrally formed row of micro-hook pads 22. The row of micro-hook pads 22 may be formed in a central portion of a planar surface 24 of the support web 20. The micro-hook pads 22 may be formed as previously disclosed with reference to figures 4 and 5.

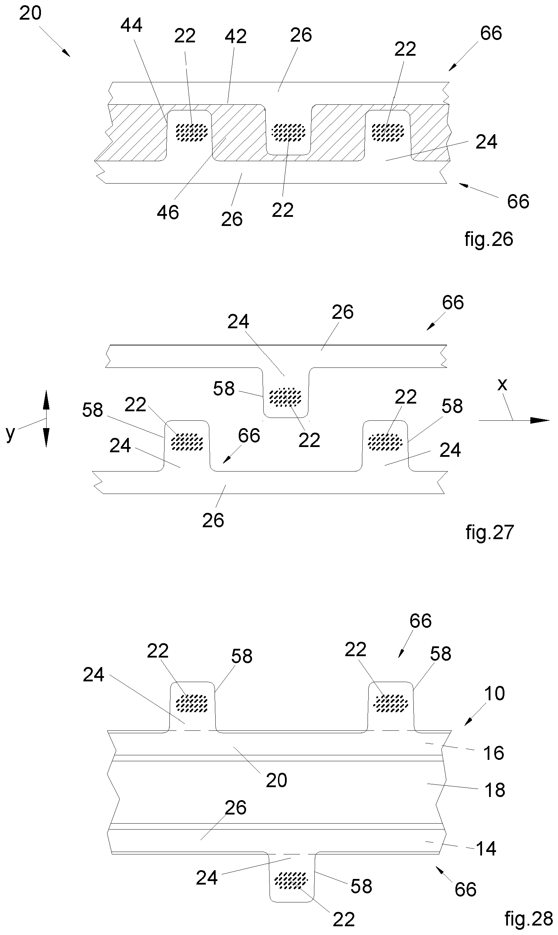

With reference to figure 26, the method further comprises a cutting step wherein the continuous support web 20 is cut along two continuous undulated cutting lines 42, 44 passing between each pair of adjacent micro-hook pads 22. The two continuous undulated cutting lines 42, 44 define a continuous undulated strip 46, shown by hatched lines in figure 26, comprised between the two cutting lines 42, 44 which is disposed as waste. In a possible embodiment, the continuous support web 20 may be cut along only one continuous undulated cutting line.

With reference to figure 27, after the cut of the support web 20 along the two continuous undulated cutting lines 42, 44 two separate continuous tab chains 66, are formed, which are offset from each other in the longitudinal direction X. The two continuous tab chains 66, comprise respective continuous longitudinal edge portions 26, and respective tabs 58 projecting from the respective continuous longitudinal edge portions 26 in the transversal direction Y. Each tab 58 has a respective micro-hook pad 22 integrally formed on a respective planar surface 24. Each micro-hook pad 22 is completely surrounded by a portion of the planar surface 24. In each continuous tab chain 66 the respective tabs 58 are spaced apart from each other in the longitudinal direction X.

After the cut of the support web 20 along the two continuous undulated cutting lines 42, 44 the two separate continuous tab chains 66 have the respective tabs 58 facing inward with respect to a longitudinal centre line. After the cut, the two separate continuous tab chains 66 are crossed with each other as they advance in the longitudinal direction, in order to bring the two separate continuous tab chains 66 in a position in which the respective tabs 58 face outward with respect to a longitudinal centre line.

Then, as shown in figure 28, the two continuous longitudinal edge portions 26 of the two continuous tab chains 66 are overlapped, respectively, to the first edge portion 14 and to the second edge portion 16 of the first continuous web 10.

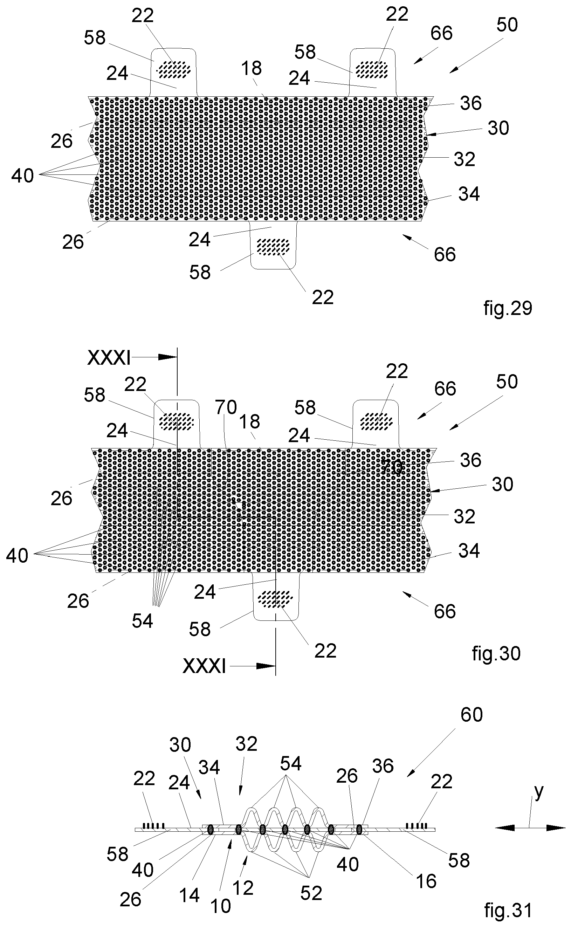

In a further step shown in figure 29, a second continuous web 30 is fed in the longitudinal direction X and is overlapped to the first continuous web 10.

The second continuous web 30 may have the same width as the first continuous web 10 and may be made of the same material (e.g. a non-woven material). The second continuous web 30 has a central portion 32 and first and second edge portions 34, 36 opposite to each other with respect to the central portion 12.

The second continuous web 30 is applied over the first continuous web 10 so that the continuous elastic tape 18 is sandwiched between the respective central portions 12, 32 of the first and second webs 10, 30. The continuous longitudinal edge portions 26 of the two continuous tab chains 66 may be sandwiched between respective first edge portions 14, 34 of the first and second continuous web 10, 30 or may be applied on the outer surface of the first or second edge portion 14, 34.

Then, the first and second web 10, 30, the elastic tape 18 and the two continuous longitudinal edge portions 26 of the two continuous tab chains 66 are joined to each other by a pattern of spot welds 40.

After the spot-welding step, a finished transversely extensible continuous elastic laminate 50 is obtained.

The tabs 58 of the two continuous tab chains 66 may be folded over the second web 30 along respective folding lines parallel to the longitudinal axis X, so that the micro-hook pads 22 are releasably fastened to corresponding portions of the second web 30.

With reference to figures 30 and 31, when the vacuum suction which holds the elastic tape 18 stretched in the transversal direction Y is released, the elastic tape 18 contracts in the transversal direction Y. The transversal contraction of the elastic tape 18 forms longitudinal pleats 52, 54 on the central portions 12, 32 of the first and second continuous web 10, 30. The edge portions 14, 34 and 16, 36 of the first and second continuous webs 10, 30 which extend transversally beyond the elastic tape 18 remain non-pleated.

With reference to figures 30 and 31, the transversely extensible continuous elastic laminate 50 obtained by the method previously disclosed comprises a first and second continuous web 10, 30 overlapped to each other and extending along a longitudinal direction X. The first and second continuous web 10, 30 have respective pleated central portions 12, 32 having longitudinal pleats 52, 54 and respective first and second non-pleated edge portions 14, 34 and 16, 36.

A continuous elastic tape 18 elastically extensible in a transverse direction Y is sandwiched between the pleated central portions 12, 32 of the first and second continuous webs 10, 30 and joined thereto by a pattern of spot welds 40.

The transversely extensible continuous elastic laminate 50 comprises a first and a second tab chain 66 of non-woven material having respective continuous longitudinal edge portions 26 and respective rows of tabs 58 having respective micro-hook pads 22 integrally formed with the non-woven material of the tabs 58.

In each tab chain 66 the respective tabs 58 are spaced apart from each other in the longitudinal direction X. The tabs 58 of the two tab chains 66 are offset with respect to each other.

The continuous longitudinal edge portions 26 of the first and second tab chains 66 are joined to the respective first and second non-pleated edge portions 14, 34 and 16, 36 of the first and second continuous web 10, 30 by said pattern of spot welds 40. The pattern of spot welds 40 may be reinforced in the areas joining the continuous longitudinal edge portions 26 of the first and second tab chain 66 and the respective first and second non-pleated edge portions 14, 34 and 16, 36 of the first and second continuous web 10, 30.

In a possible embodiment, the micro-hook pads 22 of the first and second tab chain 66 may have respective hooks oriented in opposite direction to each other.

The transversely extensible continuous elastic laminate 50 may be cut along cutting lines 70 (figure 30) for forming individual elastic side panels 60, as previously disclosed.

An absorbent sanitary article may have an absorbent central body and two or four elastic side panels 60 as previously described fixed to two opposite longitudinal edges of said central body.

As compared to the prior art disclosed in EP-A-3213728, the elastic side panels 60 according to the present invention have an increased resistance and are less exposed to the risk of breaks because the tabs 58 are connected to the webs 10, 30 along the whole length of the webs 10, 30.

In use, the tabs 58 have a soft feeling to the touch because the micro-hook pads 22 are completely surrounded by a portion of non-woven material which is softer than the relatively rigid thermoplastic material forming the micro-hook pads of the prior art.

The method for manufacturing the transversely extensible continuous elastic laminate 50 does not require the application of discrete tabs on the edges of the elastic laminate. The tabs with the respective micro-hook pads 22 are formed by applying and cutting a continuous web. This involves considerable advantages in that there is no need for complex and expensive repitch devices for applying discrete elements at a constant pitch.

Of course, without prejudice to the principle of the invention, the details of construction and the embodiments can be varied, even significantly, with respect to those illustrated here without departing from the scope of the invention as defined by the following claims.

## Claims

1. A transversely extensible continuous elastic laminate (50) comprising:
- a first and a second continuous web (10, 30) extending along a longitudinal direction (X), having respective pleated central portions (12, 32) having pleats (52, 54) parallel to said longitudinal direction (X) and respective first and second non-pleated edge portions (14, 34, 16, 36) on opposite sides of said pleated central portions (12, 32),
- a continuous elastic tape (18) extending in said longitudinal direction (X), and sandwiched between said pleated central portions (12, 32) of said first and second web (10, 30), wherein said elastic tape (18) is elastically stretchable in a transversal direction (Y) transversal to said longitudinal direction (X), and wherein said continuous elastic tape (18) is joined to the first and second web (10, 30) by a pattern of spot welds (40), and
- at least one row of tabs (58) protruding in said transversal direction (Y) beyond at least one of said non-pleated edge portions (14, 34, 16, 36), said tabs (58) being made of a non-woven material and having respective micro-hook pads (22) integrally formed in the non-woven material forming said tabs (58), wherein each of said micro-hook pads (22) is completely surrounded by a portion of a planar surface (24) of the respective tab (58).

2. The transversely extensible continuous elastic laminate of claim 1, wherein said at least one row of tabs (58) includes a continuous edge portion (26) of a non-woven material extending in said longitudinal direction (X) and joined to one of said non-pleated edge portions (14, 34, 16, 36) of said first and second web (10, 30) by said pattern of spot welds (40), said tabs (58) transversely extending from said continuous edge portion (26), being connected to each other by said continuous edge portion (26) and being spaced apart from each other in said longitudinal direction (X).

3. The transversely extensible continuous elastic laminate of claim 1, wherein said at least one row of tabs (58) is formed by a portion of said first or second continuous web (10, 30).

4. The transversely extensible continuous elastic laminate of claim 1, comprising a first and a second tab chain (66) of non-woven material having respective continuous longitudinal edge portions (26) joined to respective first and second non-pleated edge portions (14, 16, 34, 36) of the first and second continuous web (10, 30) and fixed thereto by said pattern of spot welds (40), and respective tabs (58) extending from the respective continuous longitudinal edge portions (26).

5. The transversely extensible continuous elastic laminate of claim 4, wherein in each of said continuous tab chains (66) the respective tabs (58) are spaced apart from each other in said longitudinal direction (X) and wherein the tabs (58) of the two continuous tab chains (66) are offset with respect to each other.

6. An elastic side panel for absorbent sanitary articles, comprising:
- a first and a second web (10, 30), having respective pleated central portions (12, 32) having pleats (52, 54) parallel to a first direction (X), and respective first and second non-pleated edge portions (14, 34, 16, 36) on opposite sides of said pleated central portions (12, 32),
- an elastic tape (18) sandwiched between said pleated central portions (12, 32) of said first and second web (10, 30), wherein said elastic tape (18) is elastically stretchable in a second direction (Y) transversal to said first direction (X), wherein said elastic tape (18) is joined to the first and second web (10) by a pattern of spot welds (40), and
- a tab (58) protruding in said transversal direction (Y) beyond at least one of said non-pleated edge portions (14, 34, 16, 36), said tab (58) being made of a non-woven material and having a respective micro-hook pad (22) integrally formed in the non-woven material forming said tab (58), wherein said micro-hook pad (22) is completely surrounded by a portion of a planar surface (24) of the tab (58).

7. The elastic side panel of claim 6, comprising a support web (20) of a non-woven material including an edge portion (26) joined to one of said non-pleated edge portions (14, 34, 16, 36) of said first and second web (10, 30) and fixed thereto by said pattern of spot welds (40), said edge portion (26) having in said first direction (X) the same length as said non-pleated edge portion (14, 34, 16, 36), the support web (20) including an integral tab (58) extending from said edge portion (26).

8. The elastic side panel of claim 6, wherein said tab (58) is integrally formed with said first and/or second web (10, 30).

9. An absorbent sanitary article comprising an absorbent central body and two or four elastic side panels (60) according to any of claims 6-8 fixed to two opposite longitudinal edges of said central body.

10. A method for producing a continuous transversely extensible elastic laminate (50) comprising:
- feeding in a longitudinal direction (X) two first continuous webs (10), the two first continuous webs (10) being spaced from each other in a transverse direction (Y) and having respective central portions (12) and respective first and second edge portions (14, 16) on opposite sides of the respective central portions (12),
- feeding in said longitudinal direction (X) two continuous elastic tapes (18),
- elastically stretching said two continuous elastic tapes (18) in a transverse direction (Y) transversal to said longitudinal direction (X), and applying said two continuous elastic tapes to respective central portions (12) of said two first continuous webs (10) while stretched in said transversal direction (Y),
- feeding in said longitudinal direction (X) a continuous support web (20) of a non-woven material having two continuous edge portions (26), the continuous support web (20) having a row of micro-hook pads (22) integrally formed in the non-woven material forming said support web (20),
- applying said two continuous longitudinal edge portions (26) of said support web (20) to respective first edge portions (14) of said two first continuous webs (10),
- feeding in said longitudinal direction (X)two second continuous webs (30) spaced from each other in said transverse direction (Y) and having respective central portions (32) and respective first and second edge portions (34, 36) on opposite sides of the respective central portions (32),
- applying said two second continuous webs (30) to respective first continuous webs (10) and sandwiching said two continuous elastic tapes (18) between respective central portions (12, 32) of said first and second webs (10, 30),
- joining to each other said two first and second webs (10, 30), said two elastic tapes (18) and the edge portions (26) of the support web (20) by a pattern of spot welds (40) while said two elastic tapes (18) are stretched in said transversal direction (Y), and
- cutting the support web (20) along at least one continuous undulated cutting line (42) passing between each pair of adjacent micro-hook pads (22) so as to form two separate continuous transversely extensible elastic laminates (50),
wherein each of said continuous transversely extensible elastic laminates (50) has a respective pleated central portion (12, 32) having pleats (52, 54) parallel to said longitudinal direction (X) and respective first and second non-pleated edge portions (14, 34, 16, 36) on opposite sides of said pleated central portions (12, 32).

11. A method for producing a continuous transversely extensible elastic laminate (50) comprising:
- feeding in a longitudinal direction (X) a first continuous web (10),
- feeding in said longitudinal direction (X) two continuous elastic tapes (18) parallel to each other and spaced from each other in a transverse direction (Y) transversal to said longitudinal direction (X),
- elastically stretching said two continuous elastic tapes (18) in a transverse direction (Y), and applying said two continuous elastic tapes (18) to respective portions of said first continuous web (10) while stretched in said transversal direction (Y),
- feeding a second continuous web (30) of a non-woven material in said longitudinal direction (X), the second continuous web (30) having a row of micro-hook pads (22) integrally formed in the non-woven material forming said second continuous web (30),
- applying said second continuous web (30) to the first continuous web (10) and sandwiching said two continuous elastic tapes (18) between respective portions of said first and second webs (10, 30),
- joining to each other said first and second web (10, 30) and said two elastic tapes (18) by a pattern of spot welds (40) while said two elastic tapes (18) are stretched in said transversal direction (Y),
- cutting said first and second webs (10, 30) along at least one continuous undulated cutting line (42) passing between each pair of adjacent micro-hook pads (22) so as to form two separate continuous transversely extensible elastic laminates (50),
wherein each of said continuous transversely extensible elastic laminates (50) has a respective pleated central portion (12, 32) having pleats (52, 54) parallel to said longitudinal direction (X) and respective first and second non-pleated edge portions (14, 34, 16, 36) on opposite sides of said pleated central portions (12, 32).

12. A method for producing a continuous transversely extensible elastic laminate (50) comprising:
- feeding in a longitudinal direction (X) a first continuous web (10) having a central portion (12) and first and second edge portions (14, 16) on opposite sides of the central portion (12),
- feeding in said longitudinal direction (X) a continuous elastic tape (18),
- elastically stretching said continuous elastic tape (18) in a transverse direction (Y) transversal to said longitudinal direction (X), and applying said continuous elastic tape to the central portion of said first continuous web (10) while stretched in said transversal direction (Y),
- feeding in said longitudinal direction (X), a continuous support web (20) of a non-woven material having a row of micro-hook pads (22) integrally formed in the non-woven material forming said support web (20),
- cutting the continuous support web (20) along at least one continuous undulated cutting line (42, 44) passing between each pair of adjacent micro-hook pads (22) so as to form two separate tab chains (66) having respective continuous longitudinal edge portions (26) and respective tabs (58) having respective integral micro-hook pads (22),
- applying said two continuous longitudinal edge portions (26) of said continuous tab chains (66) to respective first edge portions (14, 16) of said first continuous web (10),
- feeding in said longitudinal direction (X) a second continuous web (30) having a central portion (32) and first and second edge portions (34, 36) on opposite sides of the central portion (52),
- applying said second continuous web (30) to the first continuous web (10) and sandwiching said continuous elastic tape (18) between respective central portions (12, 32) of said first and second webs (10, 30), and
- joining to each other said first and second webs (10, 30), said elastic tape (18) and said two continuous longitudinal edge portions (26) of said continuous tab chains (66) by a pattern of spot welds (40), so as to form a continuous transversely extensible elastic laminate (50),
wherein said continuous transversely extensible elastic laminate (50) has a pleated central portion (12, 32) having pleats (52, 54) parallel to said longitudinal direction (X) and first and second non-pleated edge portions (14, 34, 16, 36) on opposite sides of said pleated central portions (12, 32).

13. The method of claim 10 or 11, wherein said at least one continuous undulated cutting line (42, 44) forms in each of said continuous transversely extensible elastic laminates (50) a row of tabs (58) connected to each other by a respective continuous longitudinal edge portion (26) and spaced apart from each other in said longitudinal direction (X).

14. The method of any of claims 10, 11, or 12, wherein said row of micro-hook pads (22) is integrally formed in said non-woven material by locally liquifying or fluidifying the non-woven material and inserting the liquified or fluidified non-woven material into cavities of a molding roller (29).

15. The method of claim 14, wherein locally liquifying or fluidifying the non-woven material is obtained by subjecting the non-woven material to ultrasonic compression or to thermomechanical compression.

## Patentansprüche

1. Ein quer dehnbares kontinuierliches elastisches Laminat (50), umfassend:
- eine erste und eine zweite kontinuierliche Bahn (10, 30), die sich entlang einer Längsrichtung (X) erstrecken, mit jeweiligen gefalteten Mittelabschnitten (12, 32), die Falten (52, 54) parallel zu der genannten Längsrichtung (X) aufweisen, und jeweiligen ersten und zweiten ungefalteten Randabschnitten (14, 34, 16, 36) auf gegenüberliegenden Seiten der genannten gefalteten Mittelabschnitte (12, 32),
- ein kontinuierliches elastisches Band (18), das sich in der genannten Längsrichtung (X) erstreckt und zwischen den genannten gefalteten Mittelabschnitten (12, 32) der genannten ersten und zweiten Bahn (10, 30) eingebettet ist, wobei das genannte elastische Band (18) in einer Querrichtung (Y), die quer zu der genannten Längsrichtung (X) verläuft, elastisch dehnbar ist, und wobei das genannte kontinuierliche elastische Band (18) mit der ersten und zweiten Bahn (10, 30) durch ein Muster von Punktschweißungen (40) verbunden ist, und
- mindestens eine Reihe von Laschen (58), die in der genannten Querrichtung (Y) über mindestens einen der genannten ungefalteten Randabschnitte (14, 34, 16, 36) hinausragen, wobei die genannten Laschen (58) aus einem Vliesmaterial bestehen und jeweilige Mikrohaken-Pads (22) aufweisen, die integral in dem die genannten Laschen (58) bildenden Vliesmaterial ausgebildet sind, wobei jedes der genannten Mikrohaken-Pads (22) vollständig von einem Abschnitt einer planaren Oberfläche (24) der jeweiligen Lasche (58) umgeben ist.

2. Das quer dehnbare kontinuierliche elastische Laminat nach Anspruch 1, wobei die genannte mindestens eine Reihe von Laschen (58) einen kontinuierlichen Randabschnitt (26) aus einem Vliesmaterial einschließt, der sich in der genannten Längsrichtung (X) erstreckt und mit einem der genannten ungefalteten Randabschnitte (14, 34, 16, 36) der genannten ersten und zweiten Bahn (10, 30) durch das genannte Muster von Punktschweißungen (40) verbunden ist, wobei die genannten Laschen (58) sich quer von dem genannten kontinuierlichen Randabschnitt (26) erstrecken, durch den genannten kontinuierlichen Randabschnitt (26) miteinander verbunden sind und in der genannten Längsrichtung (X) voneinander beabstandet sind.

3. Das quer dehnbare kontinuierliche elastische Laminat nach Anspruch 1, wobei die genannte mindestens eine Reihe von Laschen (58) durch einen Abschnitt der genannten ersten oder zweiten kontinuierlichen Bahn (10, 30) gebildet ist.

4. Das quer dehnbare kontinuierliche elastische Laminat nach Anspruch 1, umfassend eine erste und eine zweite Laschenkette (66) aus Vliesmaterial mit jeweiligen kontinuierlichen Längsrandabschnitten (26), die mit jeweiligen ersten und zweiten ungefalteten Randabschnitten (14, 16, 34, 36) der ersten und zweiten kontinuierlichen Bahn (10, 30) verbunden und daran durch das genannte Muster von Punktschweißungen (40) befestigt sind, und jeweiligen Laschen (58), die sich von den jeweiligen kontinuierlichen Längsrandabschnitten (26) erstrecken.

5. Das quer dehnbare kontinuierliche elastische Laminat nach Anspruch 4, wobei in jeder der genannten kontinuierlichen Laschenketten (66) die jeweiligen Laschen (58) in der genannten Längsrichtung (X) voneinander beabstandet sind und wobei die Laschen (58) der zwei kontinuierlichen Laschenketten (66) zueinander versetzt sind.

6. Eine elastische Seitenklappe für saugfähige Hygieneartikel, umfassend:
- eine erste und eine zweite Bahn (10, 30) mit jeweiligen gefalteten Mittelabschnitten (12, 32), die Falten (52, 54) parallel zu einer ersten Richtung (X) aufweisen, und jeweiligen ersten und zweiten ungefalteten Randabschnitten (14, 34, 16, 36) auf gegenüberliegenden Seiten der genannten gefalteten Mittelabschnitte (12, 32),
- ein elastisches Band (18), das zwischen den genannten gefalteten Mittelabschnitten (12, 32) der genannten ersten und zweiten Bahn (10, 30) eingebettet ist, wobei das genannte elastische Band (18) in einer zweiten Richtung (Y), die quer zu der genannten ersten Richtung (X) verläuft, elastisch dehnbar ist, wobei das genannte elastische Band (18) mit der ersten und zweiten Bahn (10) durch ein Muster von Punktschweißungen (40) verbunden ist, und
- eine Lasche (58), die in der genannten Querrichtung (Y) über mindestens einen der genannten ungefalteten Randabschnitte (14, 34, 16, 36) hinausragt, wobei die genannte Lasche (58) aus einem Vliesmaterial besteht und ein jeweiliges Mikrohaken-Pad (22) aufweist, das integral in dem die genannte Lasche (58) bildenden Vliesmaterial ausgebildet ist, wobei das genannte Mikrohaken-Pad (22) vollständig von einem Abschnitt einer planaren Oberfläche (24) der Lasche (58) umgeben ist.

7. Die elastische Seitenklappe nach Anspruch 6, umfassend eine Trägerbahn (20) aus einem Vliesmaterial, die einen Randabschnitt (26) einschließt, der mit einem der genannten ungefalteten Randabschnitte (14, 34, 16, 36) der genannten ersten und zweiten Bahn (10, 30) verbunden und daran durch das genannte Muster von Punktschweißungen (40) befestigt ist, wobei der genannte Randabschnitt (26) in der genannten ersten Richtung (X) die gleiche Länge wie der genannte ungefaltete Randabschnitt (14, 34, 16, 36) aufweist, wobei die Trägerbahn (20) eine integrale Lasche (58) einschließt, die sich von dem genannten Randabschnitt (26) erstreckt.

8. Die elastische Seitenklappe nach Anspruch 6, wobei die genannte Lasche (58) integral mit der genannten ersten und/oder zweiten Bahn (10, 30) ausgebildet ist.

9. Ein saugfähiger Hygieneartikel, umfassend einen saugfähigen Mittelkörper und zwei oder vier elastische Seitenklappen (60) nach einem der Ansprüche 6-8, die an zwei gegenüberliegenden Längsrändern des genannten Mittelkörpers befestigt sind.

10. Ein Verfahren zur Herstellung eines kontinuierlichen quer dehnbaren elastischen Laminats (50), umfassend:
- Zuführen von zwei ersten kontinuierlichen Bahnen (10) in einer Längsrichtung (X), wobei die zwei ersten kontinuierlichen Bahnen (10) in einer Querrichtung (Y) voneinander beabstandet sind und jeweilige Mittelabschnitte (12) und jeweilige erste und zweite Randabschnitte (14, 16) auf gegenüberliegenden Seiten der jeweiligen Mittelabschnitte (12) aufweisen,
- Zuführen von zwei kontinuierlichen elastischen Bändern (18) in der genannten Längsrichtung (X),
- elastisches Dehnen der genannten zwei kontinuierlichen elastischen Bänder (18) in einer Querrichtung (Y), die quer zu der genannten Längsrichtung (X) verläuft, und Aufbringen der genannten zwei kontinuierlichen elastischen Bänder auf jeweilige Mittelabschnitte (12) der genannten zwei ersten kontinuierlichen Bahnen (10), während sie in der genannten Querrichtung (Y) gedehnt sind,
- Zuführen einer kontinuierlichen Trägerbahn (20) aus einem Vliesmaterial mit zwei kontinuierlichen Randabschnitten (26) in der genannten Längsrichtung (X), wobei die kontinuierliche Trägerbahn (20) eine Reihe von Mikrohaken-Pads (22) aufweist, die integral in dem die genannte Trägerbahn (20) bildenden Vliesmaterial ausgebildet sind,
- Aufbringen der genannten zwei kontinuierlichen Längsrandabschnitte (26) der genannten Trägerbahn (20) auf jeweilige erste Randabschnitte (14) der genannten zwei ersten kontinuierlichen Bahnen (10),
- Zuführen von zwei zweiten kontinuierlichen Bahnen (30) in der genannten Längsrichtung (X), die in der genannten Querrichtung (Y) voneinander beabstandet sind und jeweilige Mittelabschnitte (32) und jeweilige erste und zweite Randabschnitte (34, 36) auf gegenüberliegenden Seiten der jeweiligen Mittelabschnitte (32) aufweisen,
- Aufbringen der genannten zwei zweiten kontinuierlichen Bahnen (30) auf jeweilige erste kontinuierliche Bahnen (10) und Einbetten der genannten zwei kontinuierlichen elastischen Bänder (18) zwischen jeweiligen Mittelabschnitten (12, 32) der genannten ersten und zweiten Bahnen (10, 30),
- Verbinden der genannten zwei ersten und zweiten Bahnen (10, 30), der genannten zwei elastischen Bänder (18) und der Randabschnitte (26) der Trägerbahn (20) miteinander durch ein Muster von Punktschweißungen (40), während die genannten zwei elastischen Bänder (18) in der genannten Querrichtung (Y) gedehnt sind, und
- Schneiden der Trägerbahn (20) entlang mindestens einer kontinuierlichen wellenförmigen Schnittlinie (42), die zwischen jedem Paar benachbarter Mikrohaken-Pads (22) verläuft, um zwei separate kontinuierliche quer dehnbare elastische Laminate (50) zu bilden,
wobei jedes der genannten kontinuierlichen quer dehnbaren elastischen Laminate (50) einen jeweiligen gefalteten Mittelabschnitt (12, 32) mit Falten (52, 54) parallel zu der genannten Längsrichtung (X) und jeweilige erste und zweite ungefaltete Randabschnitte (14, 34, 16, 36) auf gegenüberliegenden Seiten der genannten gefalteten Mittelabschnitte (12, 32) aufweist.

11. Ein Verfahren zur Herstellung eines kontinuierlichen quer dehnbaren elastischen Laminats (50), umfassend:
- Zuführen einer ersten kontinuierlichen Bahn (10) in einer Längsrichtung (X),
- Zuführen von zwei kontinuierlichen elastischen Bändern (18) in der genannten Längsrichtung (X), die parallel zueinander und in einer Querrichtung (Y), die quer zu der genannten Längsrichtung (X) verläuft, voneinander beabstandet sind,
- elastisches Dehnen der genannten zwei kontinuierlichen elastischen Bänder (18) in einer Querrichtung (Y) und Aufbringen der genannten zwei kontinuierlichen elastischen Bänder (18) auf jeweilige Abschnitte der genannten ersten kontinuierlichen Bahn (10), während sie in der genannten Querrichtung (Y) gedehnt sind,
- Zuführen einer zweiten kontinuierlichen Bahn (30) aus einem Vliesmaterial in der genannten Längsrichtung (X), wobei die zweite kontinuierliche Bahn (30) eine Reihe von Mikrohaken-Pads (22) aufweist, die integral in dem die genannte zweite kontinuierliche Bahn (30) bildenden Vliesmaterial ausgebildet sind,
- Aufbringen der genannten zweiten kontinuierlichen Bahn (30) auf die erste kontinuierliche Bahn (10) und Einbetten der genannten zwei kontinuierlichen elastischen Bänder (18) zwischen jeweiligen Abschnitten der genannten ersten und zweiten Bahnen (10, 30),
- Verbinden der genannten ersten und zweiten Bahn (10, 30) und der genannten zwei elastischen Bänder (18) miteinander durch ein Muster von Punktschweißungen (40), während die genannten zwei elastischen Bänder (18) in der genannten Querrichtung (Y) gedehnt sind,
- Schneiden der genannten ersten und zweiten Bahnen (10, 30) entlang mindestens einer kontinuierlichen wellenförmigen Schnittlinie (42), die zwischen jedem Paar benachbarter Mikrohaken-Pads (22) verläuft, um zwei separate kontinuierliche quer dehnbare elastische Laminate (50) zu bilden,
wobei jedes der genannten kontinuierlichen quer dehnbaren elastischen Laminate (50) einen jeweiligen gefalteten Mittelabschnitt (12, 32) mit Falten (52, 54) parallel zu der genannten Längsrichtung (X) und jeweilige erste und zweite ungefaltete Randabschnitte (14, 34, 16, 36) auf gegenüberliegenden Seiten der genannten gefalteten Mittelabschnitte (12, 32) aufweist.

12. Ein Verfahren zur Herstellung eines kontinuierlichen quer dehnbaren elastischen Laminats (50), umfassend:
- Zuführen einer ersten kontinuierlichen Bahn (10) in einer Längsrichtung (X), die einen Mittelabschnitt (12) und erste und zweite Randabschnitte (14, 16) auf gegenüberliegenden Seiten des Mittelabschnitts (12) aufweist,
- Zuführen eines kontinuierlichen elastischen Bandes (18) in der genannten Längsrichtung (X),
- elastisches Dehnen des genannten kontinuierlichen elastischen Bandes (18) in einer Querrichtung (Y), die quer zu der genannten Längsrichtung (X) verläuft, und Aufbringen des genannten kontinuierlichen elastischen Bandes auf den Mittelabschnitt der genannten ersten kontinuierlichen Bahn (10), während es in der genannten Querrichtung (Y) gedehnt ist,
- Zuführen einer kontinuierlichen Trägerbahn (20) aus einem Vliesmaterial in der genannten Längsrichtung (X), die eine Reihe von Mikrohaken-Pads (22) aufweist, die integral in dem die genannte Trägerbahn (20) bildenden Vliesmaterial ausgebildet sind,
- Schneiden der kontinuierlichen Trägerbahn (20) entlang mindestens einer kontinuierlichen wellenförmigen Schnittlinie (42, 44), die zwischen jedem Paar benachbarter Mikrohaken-Pads (22) verläuft, um zwei separate Laschenketten (66) zu bilden, die jeweilige kontinuierliche Längsrandabschnitte (26) und jeweilige Laschen (58) mit jeweiligen integralen Mikrohaken-Pads (22) aufweisen,
- Aufbringen der genannten zwei kontinuierlichen Längsrandabschnitte (26) der genannten kontinuierlichen Laschenketten (66) auf jeweilige erste Randabschnitte (14, 16) der genannten ersten kontinuierlichen Bahn (10),
- Zuführen einer zweiten kontinuierlichen Bahn (30) in der genannten Längsrichtung (X), die einen Mittelabschnitt (32) und erste und zweite Randabschnitte (34, 36) auf gegenüberliegenden Seiten des Mittelabschnitts (32) aufweist,
- Aufbringen der genannten zweiten kontinuierlichen Bahn (30) auf die erste kontinuierliche Bahn (10) und Einbetten des genannten kontinuierlichen elastischen Bandes (18) zwischen jeweiligen Mittelabschnitten (12, 32) der genannten ersten und zweiten Bahnen (10, 30), und
- Verbinden der genannten ersten und zweiten Bahnen (10, 30), des genannten elastischen Bandes (18) und der genannten zwei kontinuierlichen Längsrandabschnitte (26) der genannten kontinuierlichen Laschenketten (66) miteinander durch ein Muster von Punktschweißungen (40), um ein kontinuierliches quer dehnbares elastisches Laminat (50) zu bilden,
wobei das genannte kontinuierliche quer dehnbare elastische Laminat (50) einen gefalteten Mittelabschnitt (12, 32) mit Falten (52, 54) parallel zu der genannten Längsrichtung (X) und erste und zweite ungefaltete Randabschnitte (14, 34, 16, 36) auf gegenüberliegenden Seiten der genannten gefalteten Mittelabschnitte (12, 32) aufweist.

13. Das Verfahren nach Anspruch 10 oder 11, wobei die genannte mindestens eine kontinuierliche wellenförmige Schnittlinie (42, 44) in jedem der genannten kontinuierlichen quer dehnbaren elastischen Laminate (50) eine Reihe von Laschen (58) bildet, die durch einen jeweiligen kontinuierlichen Längsrandabschnitt (26) miteinander verbunden und in der genannten Längsrichtung (X) voneinander beabstandet sind.

14. Das Verfahren nach einem der Ansprüche 10, 11 oder 12, wobei die genannte Reihe von Mikrohaken-Pads (22) integral in dem genannten Vliesmaterial ausgebildet wird, indem das Vliesmaterial lokal verflüssigt oder verfließend gemacht wird und das verflüssigte oder verfließend gemachte Vliesmaterial in Hohlräume einer Formwalze (29) eingebracht wird.

15. Das Verfahren nach Anspruch 14, wobei das lokale Verflüssigen oder Verfließendmachen des Vliesmaterials erreicht wird, indem das Vliesmaterial einer Ultraschallkompression oder einer thermomechanischen Kompression ausgesetzt wird.

## Revendications

1. Stratifié élastique continu extensible transversalement (50) comprenant :
- une première et une deuxième bande continue (10, 30) s'étendant le long d'une direction longitudinale (X), ayant des parties centrales plissées respectives (12, 32) présentant des plis (52, 54) parallèles à ladite direction longitudinale (X) et des première et deuxième parties de bord non plissées respectives (14, 34, 16, 36) sur des côtés opposés desdites parties centrales plissées (12, 32),
- une bande élastique continue (18) s'étendant dans ladite direction longitudinale (X), et prise en sandwich entre lesdites parties centrales plissées (12, 32) de ladite première et de ladite deuxième bande (10, 30), dans lequel ladite bande élastique (18) est élastiquement étirable dans une direction transversale (Y) transversale à ladite direction longitudinale (X), et dans lequel ladite bande élastique continue (18) est jointe à la première et à la deuxième bande (10, 30) par un motif de soudures par points (40), et
- au moins une rangée de pattes (58) faisant saillie dans ladite direction transversale (Y) au-delà d'au moins une desdites parties de bord non plissées (14, 34, 16, 36), lesdites pattes (58) étant constituées d'un matériau non tissé et ayant des patins à micro-crochets respectifs (22) formés intégralement dans le matériau non tissé formant lesdites pattes (58), dans lequel chacun desdits patins à micro-crochets (22) est complètement entouré par une partie d'une surface plane (24) de la patte respective (58).

2. Stratifié élastique continu extensible transversalement selon la revendication 1, dans lequel ladite au moins une rangée de pattes (58) inclut une partie de bord continue (26) d'un matériau non tissé s'étendant dans ladite direction longitudinale (X) et jointe à une desdites parties de bord non plissées (14, 34, 16, 36) de ladite première et de ladite deuxième bande (10, 30) par ledit motif de soudures par points (40), lesdites pattes (58) s'étendant transversalement depuis ladite partie de bord continue (26), étant reliées les unes aux autres par ladite partie de bord continue (26) et étant espacées les unes des autres dans ladite direction longitudinale (X).

3. Stratifié élastique continu extensible transversalement selon la revendication 1, dans lequel ladite au moins une rangée de pattes (58) est formée par une partie de ladite première ou de ladite deuxième bande continue (10, 30).

4. Stratifié élastique continu extensible transversalement selon la revendication 1, comprenant une première et une deuxième chaîne de pattes (66) en matériau non tissé ayant des parties de bord longitudinales continues respectives (26) jointes à des première et deuxième parties de bord non plissées respectives (14, 16, 34, 36) de la première et de la deuxième bande continue (10, 30) et fixées à celles-ci par ledit motif de soudures par points (40), et des pattes respectives (58) s'étendant depuis les parties de bord longitudinales continues respectives (26).

5. Stratifié élastique continu extensible transversalement selon la revendication 4, dans lequel dans chacune desdites chaînes de pattes continues (66) les pattes respectives (58) sont espacées les unes des autres dans ladite direction longitudinale (X) et dans lequel les pattes (58) des deux chaînes de pattes continues (66) sont décalées les unes par rapport aux autres.

6. Panneau latéral élastique pour articles sanitaires absorbants, comprenant :
- une première et une deuxième bande (10, 30), ayant des parties centrales plissées respectives (12, 32) présentant des plis (52, 54) parallèles à une première direction (X), et des première et deuxième parties de bord non plissées respectives (14, 34, 16, 36) sur des côtés opposés desdites parties centrales plissées (12, 32),
- une bande élastique (18) prise en sandwich entre lesdites parties centrales plissées (12, 32) de ladite première et de ladite deuxième bande (10, 30), dans lequel ladite bande élastique (18) est élastiquement étirable dans une deuxième direction (Y) transversale à ladite première direction (X), dans lequel ladite bande élastique (18) est jointe à la première et à la deuxième bande (10) par un motif de soudures par points (40), et
- une patte (58) faisant saillie dans ladite direction transversale (Y) au-delà d'au moins une desdites parties de bord non plissées (14, 34, 16, 36), ladite patte (58) étant constituée d'un matériau non tissé et ayant un patin à micro-crochets respectif (22) formé intégralement dans le matériau non tissé formant ladite patte (58), dans lequel ledit patin à micro-crochets (22) est complètement entouré par une partie d'une surface plane (24) de la patte (58).

7. Panneau latéral élastique selon la revendication 6, comprenant une bande de support (20) d'un matériau non tissé incluant une partie de bord (26) jointe à une desdites parties de bord non plissées (14, 34, 16, 36) de ladite première et de ladite deuxième bande (10, 30) et fixée à celle-ci par ledit motif de soudures par points (40), ladite partie de bord (26) ayant dans ladite première direction (X) la même longueur que ladite partie de bord non plissée (14, 34, 16, 36), la bande de support (20) incluant une patte intégrale (58) s'étendant depuis ladite partie de bord (26).

8. Panneau latéral élastique selon la revendication 6, dans lequel ladite patte (58) est formée intégralement avec ladite première et/ou ladite deuxième bande (10, 30).

9. Article sanitaire absorbant comprenant un corps central absorbant et deux ou quatre panneaux latéraux élastiques (60) selon l'une quelconque des revendications 6 à 8 fixés à deux bords longitudinaux opposés dudit corps central.

10. Procédé de production d'un stratifié élastique continu extensible transversalement (50) comprenant :
- l'alimentation dans une direction longitudinale (X) de deux premières bandes continues (10), les deux premières bandes continues (10) étant espacées l'une de l'autre dans une direction transversale (Y) et ayant des parties centrales respectives (12) et des première et deuxième parties de bord respectives (14, 16) sur des côtés opposés des parties centrales respectives (12),
- l'alimentation dans ladite direction longitudinale (X) de deux bandes élastiques continues (18),
- l'étirement élastique desdites deux bandes élastiques continues (18) dans une direction transversale (Y) transversale à ladite direction longitudinale (X), et l'application desdites deux bandes élastiques continues sur des parties centrales respectives (12) desdites deux premières bandes continues (10) tandis qu'elles sont étirées dans ladite direction transversale (Y),
- l'alimentation dans ladite direction longitudinale (X) d'une bande de support continue (20) d'un matériau non tissé ayant deux parties de bord continues (26), la bande de support continue (20) ayant une rangée de patins à micro-crochets (22) formés intégralement dans le matériau non tissé formant ladite bande de support (20),
- l'application desdites deux parties de bord longitudinales continues (26) de ladite bande de support (20) sur des premières parties de bord respectives (14) desdites deux premières bandes continues (10),
- l'alimentation dans ladite direction longitudinale (X) de deux deuxièmes bandes continues (30) espacées l'une de l'autre dans ladite direction transversale (Y) et ayant des parties centrales respectives (32) et des première et deuxième parties de bord respectives (34, 36) sur des côtés opposés des parties centrales respectives (32),
- l'application desdites deux deuxièmes bandes continues (30) sur des premières bandes continues respectives (10) et la prise en sandwich desdites deux bandes élastiques continues (18) entre des parties centrales respectives (12, 32) desdites première et deuxième bandes (10, 30),
- la jonction les unes aux autres desdites deux première et deuxième bandes (10, 30), desdites deux bandes élastiques (18) et des parties de bord (26) de la bande de support (20) par un motif de soudures par points (40) tandis que lesdites deux bandes élastiques (18) sont étirées dans ladite direction transversale (Y), et
- la découpe de la bande de support (20) le long d'au moins une ligne de découpe ondulée continue (42) passant entre chaque paire de patins à micro-crochets adjacents (22) de manière à former deux stratifiés élastiques continus extensibles transversalement séparés (50),
dans lequel chacun desdits stratifiés élastiques continus extensibles transversalement (50) a une partie centrale plissée respective (12, 32) présentant des plis (52, 54) parallèles à ladite direction longitudinale (X) et des première et deuxième parties de bord non plissées respectives (14, 34, 16, 36) sur des côtés opposés desdites parties centrales plissées (12, 32).

11. Procédé de production d'un stratifié élastique continu extensible transversalement (50) comprenant :
- l'alimentation dans une direction longitudinale (X) d'une première bande continue (10),
- l'alimentation dans ladite direction longitudinale (X) de deux bandes élastiques continues (18) parallèles l'une à l'autre et espacées l'une de l'autre dans une direction transversale (Y) transversale à ladite direction longitudinale (X),
- l'étirement élastique desdites deux bandes élastiques continues (18) dans une direction transversale (Y), et l'application desdites deux bandes élastiques continues (18) sur des parties respectives de ladite première bande continue (10) tandis qu'elles sont étirées dans ladite direction transversale (Y),
- l'alimentation d'une deuxième bande continue (30) d'un matériau non tissé dans ladite direction longitudinale (X), la deuxième bande continue (30) ayant une rangée de patins à micro-crochets (22) formés intégralement dans le matériau non tissé formant ladite deuxième bande continue (30),
- l'application de ladite deuxième bande continue (30) sur la première bande continue (10) et la prise en sandwich desdites deux bandes élastiques continues (18) entre des parties respectives desdites première et deuxième bandes (10, 30),
- la jonction les unes aux autres desdites première et deuxième bandes (10, 30) et desdites deux bandes élastiques (18) par un motif de soudures par points (40) tandis que lesdites deux bandes élastiques (18) sont étirées dans ladite direction transversale (Y),
- la découpe desdites première et deuxième bandes (10, 30) le long d'au moins une ligne de découpe ondulée continue (42) passant entre chaque paire de patins à micro-crochets adjacents (22) de manière à former deux stratifiés élastiques continus extensibles transversalement séparés (50),
dans lequel chacun desdits stratifiés élastiques continus extensibles transversalement (50) a une partie centrale plissée respective (12, 32) présentant des plis (52, 54) parallèles à ladite direction longitudinale (X) et des première et deuxième parties de bord non plissées respectives (14, 34, 16, 36) sur des côtés opposés desdites parties centrales plissées (12, 32).

12. Procédé de production d'un stratifié élastique continu extensible transversalement (50) comprenant :
- l'alimentation dans une direction longitudinale (X) d'une première bande continue (10) ayant une partie centrale (12) et des première et deuxième parties de bord (14, 16) sur des côtés opposés de la partie centrale (12),
- l'alimentation dans ladite direction longitudinale (X) d'une bande élastique continue (18),
- l'étirement élastique de ladite bande élastique continue (18) dans une direction transversale (Y) transversale à ladite direction longitudinale (X), et l'application de ladite bande élastique continue sur la partie centrale de ladite première bande continue (10) tandis qu'elle est étirée dans ladite direction transversale (Y),
- l'alimentation dans ladite direction longitudinale (X) d'une bande de support continue (20) d'un matériau non tissé ayant une rangée de patins à micro-crochets (22) formés intégralement dans le matériau non tissé formant ladite bande de support (20),
- la découpe de la bande de support continue (20) le long d'au moins une ligne de découpe ondulée continue (42, 44) passant entre chaque paire de patins à micro-crochets adjacents (22) de manière à former deux chaînes de pattes séparées (66) ayant des parties de bord longitudinales continues respectives (26) et des pattes respectives (58) ayant des patins à micro-crochets intégrés respectifs (22),
- l'application desdites deux parties de bord longitudinales continues (26) desdites chaînes de pattes continues (66) sur des premières parties de bord respectives (14, 16) de ladite première bande continue (10),
- l'alimentation dans ladite direction longitudinale (X) d'une deuxième bande continue (30) ayant une partie centrale (32) et des première et deuxième parties de bord (34, 36) sur des côtés opposés de la partie centrale (32),
- l'application de ladite deuxième bande continue (30) sur la première bande continue (10) et la prise en sandwich de ladite bande élastique continue (18) entre des parties centrales respectives (12, 32) desdites première et deuxième bandes (10, 30), et
- la jonction les unes aux autres desdites première et deuxième bandes (10, 30), de ladite bande élastique (18) et desdites deux parties de bord longitudinales continues (26) desdites chaînes de pattes continues (66) par un motif de soudures par points (40), de manière à former un stratifié élastique continu extensible transversalement (50),
dans lequel ledit stratifié élastique continu extensible transversalement (50) a une partie centrale plissée (12, 32) présentant des plis (52, 54) parallèles à ladite direction longitudinale (X) et des première et deuxième parties de bord non plissées (14, 34, 16, 36) sur des côtés opposés desdites parties centrales plissées (12, 32).

13. Procédé selon la revendication 10 ou 11, dans lequel ladite au moins une ligne de découpe ondulée continue (42, 44) forme dans chacun desdits stratifiés élastiques continus extensibles transversalement (50) une rangée de pattes (58) reliées les unes aux autres par une partie de bord longitudinale continue respective (26) et espacées les unes des autres dans ladite direction longitudinale (X).

14. Procédé selon l'une quelconque des revendications 10, 11 ou 12, dans lequel ladite rangée de patins à micro-crochets (22) est formée intégralement dans ledit matériau non tissé en liquéfiant ou fluidifiant localement le matériau non tissé et en insérant le matériau non tissé liquéfié ou fluidifié dans des cavités d'un rouleau de moulage (29).

15. Procédé selon la revendication 14, dans lequel la liquéfaction ou fluidification locale du matériau non tissé est obtenue en soumettant le matériau non tissé à une compression ultrasonique ou à une compression thermomécanique.
